# EUROPEAN PATENT APPLICATION

(11) **EP 1 579 860 A1**
(43) Date of publication of application: **28.09.2005**
(21) Application number: 03773743.4
(22) Date of filing: 26.11.2003
(51) Int. Cl.: A61K 31/4045, A61K 7/16, A61K 38/22, A23K 1/165

(54) **BONE REGENERATION PRODUCT FOR HUMAN AND VETERINARY USE, CONTAINING MELATONIN AS ACTIVE PRINCIPLE**

(30) Priority: 31.12.2002 ES 200203054
(71) Applicant: Cutando Soriano, Antonio, 18100 Granada (ES); Gomez Moreno, Gerardo, Cajar, Granada (ES); Arana Molina, Carlos, 18007 Granada (ES); Galindo Moreno, Pablo Antonio, 18005 Granada (ES)
(72) Inventor: Cutando Soriano, Antonio, 18100 Granada (ES); Gomez Moreno, Gerardo, Cajar, Granada (ES); Arana Molina, Carlos, 18007 Granada (ES); Galindo Moreno, Pablo Antonio, 18005 Granada (ES)
(74) Representative: Munoz Garcia, Antonio
(86) International application number: PCT/ES2003/000601
(87) International publication number: WO 2004/058250

(57) **Abstract**

The invention relates to a bone regeneration product for human and veterinary use, containing melatonin as active principle, whereby toothpastes, gels, mouthwashes, bone re-absorption membranes and animal feed are provided with melatonin, in a proportion of between 0.1 % and 5 %, together with the addition of a low concentration of a colourless active antioxidant with a wide pH range, which is soluble in the medium used, substances having a synergic effect and a preservative.

## Description

### OBJECT OF THE INVENTION

The present specification refers to a bone regeneration product for human and veterinary use containing melatonin as an active principle, the evident object of which lies in allowing bone regeneration in the orofacial area and the rest of the body for its use locally and generally, both in human beings and animals, containing melatonin, taking advantage of the stimulating activity it has on the collagen fibres that participate in human bone cell synthesis, as well as its anti-inflammatory activity.

In the case of its application on human beings, it would develop bone regeneration membranes or transport through specific odontological elements such as odontological implants allowing such bone regeneration and/or osseointegration, whereas for veterinary use said melatonin effects would take advantage of both the orofacial specific route or the general route as in humans allowing bone regeneration and an increase in bone resistance through remineralisation.

### FIELD OF THE INVENTION

This invention is applicable within the industry dedicated to drug product manufacture, especially bone regenerator/anti-inflammatory products in surgical procedures, bone traumas or physiological or non-physiological bone regenerative processes in the body.

### BACKGROUND OF THE INVENTION

Melatonin is a product nocturnally secreted by the pineal gland, playing an important role in reproduction, puberty, as a potent antioxidant, as well as having oncostatic properties, it also participates in the growth hormone, in plasma corticosterone levels and in the circadian rhythm marking osteoblastic metabolism in rats, preventing phototherapy-induced hypocalcaemia.

These last actions suggest an important action of melatonin on mineral bone metabolism. These facts are reinforced by establishing relationships between melatonin secretion and osteoporosis, menopause or exercise.

"In vitro" studies performed on human jaw cell lines, have demonstrated the action melatonin exerts on human bone cells, osteoblastic cell lines, as well as on the osteogenic effect. Said action consists in a considerable proliferative effect on said cell lines, a fact which is verified by the increase in alkaline phosphatase activity, as well as an increase in the production of type I collagen synthesis.

The destructive effects produced on oral bone tissues and on the rest of the bone tissue of living being bodies by periodontopathic-type pathologies amongst others, or as a result of surgical interventions on the oral cavity, or bone restoration prosthesis insertions, create to a greater or lesser extent a bone tissue destructive-inflammatory and regenerative problem. Said bone problems bring about destructive bone damage, which leads to the disappearance or reduction in the thickness thereof and, depending on the life stage, a partial recovery or no recovery at all.

Current treatments that try to cure said problem are based on the use of bone regeneration membrane at oral cavity level, the use of animal origin bone materials, or rather taking bone samples from other parts of the body of the affected patient in an attempt to regenerate the affected bone area. Likewise, the implantation of body prosthesis and especially at an oral level, such as dental implants and the like, have the success thereof dependant on the fact of generating healthy bone around them which will allow stability and function thereof, or the creation of bone tissue replacing fibrous tissues as a consequence of infectious processes around the tooth, as occurs in pulp diseases occurring with periapical granulomas. In all these situations, the stimulating action that melatonin exerts on bone metabolism would greatly facilitate post-extraction, post-trauma and periodontopathy, bone regeneration, the joining of prosthesis to the bone, disappearance of tissues generated by infectious processes and the occurrence of healthy bone tissue, as well as general bone regeneration in certain ages or situations occurring with a melatonin deficiency.

It is known that excessive production of oxygen and nitrogen free radicals is gaining more interest every day within the main factors underlying bone injuries produced by the healthcare professional, such as complicated extractions, simple extractions, increases in bone diameter, prosthesis insertion, etc.

A local inflammatory reaction occurs simultaneously in the mouth which, amongst other things, favours the production of more free radicals.

The pathological effect of said free radicals is due to the fact that they destroy cell macromolecules such as DNA and proteins, and that they produce cell membrane oxidation.

The cell is consequently weakened, its function is altered and it dies, and along with this the regenerative capacity of the affected bone tissue is of paramount importance because said capacity can be significantly altered in certain life stages, and it can be notably increased by the action of melatonin both in different forms of local use incorporated in a carrier and by systemic route.

It is consequently desirable to dispose of a product for human and veterinary bone regeneration containing melatonin as an active principle.

However, the applicant is not aware of the current existence of a product allowing the application thereof as a bone regenerator containing melatonin.

### DESCRIPTION OF THE INVENTION

The bone regeneration product for human and veterinary use containing melatonin as an active principle proposed by the invention is made up of melatonin, which is a natural hormone produced by all living organisms, from unicellular organisms to man, including plants, and which is not therefore a synthetic but a natural product, although melatonin in pure form can however be acquired through normal chemical product suppliers, which is the manner in which it is acquired for research.

As previously set forth, said research has demonstrated its capacity for regeneration, along with its anti-inflammatory actions, and based upon these, the aim of this invention is the use of melatonin in the pathologies previously set forth, carrying melatonin in a specific form to the site of the bone injury.

More specifically, the bone regeneration product for human and veterinary use containing melatonin as an active principle object of the invention, consists of carrying the melatonin in a specific form to the site of the bone injury, this being directly impregnated on the implant or other type of prosthesis with a reabsorbable or non-reabsorbable material mixed with bone from the same patient, with gels, etc., as well as toothpastes, magistral formula mouthwashes or industrial preparations, as well as using the systemic route.

This is also valid for animals, in which the same processes described will be used, and they could also be used in their feed.

For the preparation of the toothpaste both hydrophilic and hydrophobic bases or excipients are used which are susceptible of being used in the preparation of a toothpaste, gels, mouthwashes, bone reabsorption membranes, etc.

Melatonin can be degraded by auto-oxidation or microbial enzyme action, and in order to prevent melatonin auto-oxidation the most advisable antioxidant for each case would be included in each of the aforementioned preparations.

The antioxidant will be active at low concentrations and in a wide pH range, soluble in the medium used and if possible, colourless, thermostable and non-toxic, nor irritating, nor volatile.

Amongst the most used antioxidants are sulphite, sodium bisulphite, ascorbic acid, hydroquinone, nipagin, vitamin E, vitamin A, etc., and their effectiveness may be increased by means of the use of substances with a synergic effect, such as citric and tartaric acids, EDTA, etc.

In order to prevent degradation due to microbial contamination a preservative must be added, which will not be toxic, which will be chemically stable and compatible with melatonin, amongst which are: benzoic acid, salicylic acid, ascorbic acid, essential oils, etc.

### PREFERRED EMBODIMENT OF THE INVENTION

The bone regeneration product for human and veterinary use containing melatonin as an active principle that is set forth consists in the incorporation of melatonin at a ratio ranging from 0.1% to 5% in toothpastes, gels, mouthwashes, bone reabsorption membranes and animal feeds.

As mentioned above, melatonin can be degraded by auto-oxidation or by microbial enzyme action, and in order to prevent melatonin auto-oxidation an antioxidant will be included in each of the preparations configured as toothpastes, gels, mouthwashes, bone reabsorption membranes and animal feeds, in particular a conventional antioxidant having the most suitable features for each of the cases in which it is incorporated.

The antioxidant used will be active at low concentrations and in a wide pH range, soluble in the medium used and if possible, colourless, thermostable and non-toxic, nor irritating, nor volatile.

Amongst the antioxidants that may be used are sulphite, sodium bisulphite, ascorbic acid, hydroquinone, nipagin, vitamin E, vitamin A, etc.

The antioxidant effectiveness may be increased by the use of substances with synergic effect, such as citric and tartaric acids, EDTA, etc.

In order to prevent degradation due to microbial contamination a preservative must be added, which will not be toxic, which will be chemically stable and compatible with melatonin, amongst which are: benzoic acid, salicylic acid, ascorbic acid, essential oils, etc.

## Claims

1. A bone regeneration product for human and veterinary use containing melatonin as an active principle, **characterised in that** the melatonin is incorporated in toothpastes, gels, mouthwashes, bone reabsorption membranes and animal feeds at a ratio ranging from 0.1% to 5%, with the addition of an antioxidant and a preservative, using for the toothpaste preparation both hydrophilic and hydrophobic bases or excipients susceptible of being used in the preparation of toothpastes, gels, mouthwashes, and bone reabsorption membranes.

2. A bone regeneration product for human and veterinary use containing melatonin as an active principle according to claim 1, **characterised in that** in order to prevent melatonin auto-oxidation, an antioxidant that is active at low concentrations and in a wide pH range, soluble in the medium used and if possible, colourless, thermostable and non-toxic, nor irritating, nor volatile, is included in each of the preparations.

3. A bone regeneration product for human and veterinary use containing melatonin as an active principle according to claim 1, **characterised in that** the antioxidant may be made up of sulphite, sodium bisulphite, ascorbic acid, hydroquinone, nipagin, vitamin E and vitamin A.

4. A bone regeneration product for human and veterinary use containing melatonin as an active principle according to claims 1 and 3, **characterised in that** the antioxidant effectiveness may be enhanced by means of the use of substances with a synergic effect, such as citric and tartaric acids or EDTA.

5. A bone regeneration product for human and veterinary use containing melatonin as an active principle according to claim 1, **characterised in that** the preservative prevents degradation due to microbial contamination, is non-toxic and chemically stable, compatible with melatonin and may be benzoic acid, salicylic acid, ascorbic acid or essential oils.
